(19) Europäisches Patentamt
European Patent Office
Office européen des brevets

(11) Veröffentlichungsnummer: **0 483 616 B1**

(12) **EUROPÄISCHE PATENTSCHRIFT**

(45) Veröffentlichungstag der Patentschrift: **27.12.95**

(21) Anmeldenummer: **91117801.0**

(22) Anmeldetag: **18.10.91**

(51) Int. Cl.6: **C07D 249/08**, C07D 233/60, C07D 405/08, C07D 303/08, C07D 493/10, A01N 43/653, A01N 43/50, //(C07D493/10, 303:00,303:00)

# (54) Azolylmethylspiro-2.5-octanole und diese enthaltende Fungizide

(30) Priorität: **29.10.90 DE 4034337**

(43) Veröffentlichungstag der Anmeldung:
**06.05.92 Patentblatt 92/19**

(45) Bekanntmachung des Hinweises auf die Patenterteilung:
**27.12.95 Patentblatt 95/52**

(84) Benannte Vertragsstaaten:
**AT BE CH DE DK ES FR GB GR IT LI NL SE**

(56) Entgegenhaltungen:
**EP-A- 0 378 953**
**EP-A- 0 433 780**

**MONATSHEFTE FüR CHEMIE Bd. 116, Nr. 10, 1985, WIEN Seiten 1221 - 1226; HARALD BERNHARD ET. AL.: 'Zur Stereochemie der Vitamin d3-Epoxide. Röngenstrukturanalyse einer 5,6,7,8-10,19-Triepoxidverbindung.'**

(73) Patentinhaber: **BASF Aktiengesellschaft**
**Carl-Bosch-Strasse 38**
**D-67063 Ludwigshafen (DE)**

(72) Erfinder: **Seele, Rainer, Dr.**
**Leiblstrasse 3**
**W-6701 Fussgoenheim (DE)**
Erfinder: **Goetz, Norbert, Dr.**
**Schoefferstrasse 25**
**W-6520 Worms 1 (DE)**
Erfinder: **Lorenz, Gisela, Dr.**
**Erlenweg 13**
**W-6730 Neustadt (DE)**
Erfinder: **Ammermann, Eberhard, Dr.**
**Sachsenstrasse 3**
**W-6700 Ludwigshafen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 483 616 B1

## Beschreibung

Die vorliegende Erfindung betrifft neue Azolverbindungen, Verfahren zu ihrer Herstellung und diese enthaltende Fungizide.

Es ist bekannt, E-1-[1-(4-Chlorphenyl)-1-hydroxy-2-(1,2,4-triazol-1-yl)-1-ethyl]-2-phenyl-cyclopropan (EP 212605) oder 2-(4-Chlorbenzyliden)-6,6-dimethyl-1-(1H-1,2,4-triazol-1-yl-methyl)1-hexanol (EP 378 953) als Fungizide zu verwenden. Die fungiziden Wirkungen sind jedoch nicht in allen Fällen befriedigend.

Der Erfindung lag daher die Aufgabe zugrunde, neue Azolverbindungen mit besseren fungiziden Wirkungen bereitzustellen.

Es wurde nun gefunden, daß Verbindungen der Formel I

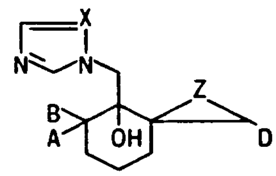

in welcher A und B jeweils für ein Wasserstoffatom oder eine $C_1$- bis $C_4$-Alkylgruppe stehen;

D

$C_1$- bis $C_8$-Alkyl, $C_3$- bis $C_6$-Cycloalkyl, $C_5$- bis $C_8$-Cycloalkenyl, Biphenylyl, Naphthyl, Heteroaryl, oder Phenyl bedeutet, wobei jeder dieser Reste einfach bis dreifach durch Halogen, Nitro, Phenoxy, Amino, $C_1$- bis $C_4$-Alkyl, $C_1$- bis $C_4$-Alkoxy oder $C_1$- bis $C_4$-Halogenalkyl substituiert sein kann

Z

den Rest $CH_2$ oder O bedeutet,

X

den Rest CH oder N bedeutet,

sowie deren pflanzenverträgliche Säureadditionssalze und Metallkomplexe eine bessere fungizide Wirkung haben als bekannte Azolderivate.

Außerdem betrifft die Erfindung Verfahren zur Herstellung der neuen Verbindungen, dabei verwendete Zwischenprodukte, fungizide Mittel, welche die erfindungsgemäßen Azolylmethylcyclohexanole als wirksame Substanzen enthalten, sowie Verfahren zur Bekämpfung von Pilzen mit Hilfe dieser Verbindungen.

Die Azolylmethylcyclohexanole der allgemeinen Formel I erhält man i.a. in Form von Racematen bzw. Diastereomerengemischen. Diese Isomeren lassen sich in üblicher Weise, beispielsweise aufgrund ihrer Löslichkeit oder der ihrer Salze oder auch durch Säulenchromatographie, trennen und in reiner Form isolieren. Aus einem solchen isolierten Diastereomeren kann man mit bekannten Methoden einheitliche Enantiomere erhalten.

Die Erfindung umfaßt sowohl die einzelnen Diastereomeren bzw. Enantiomeren als auch die bei der Synthese anfallenden Gemische und diese enthaltende Fungizide.

Bevorzugt sind diejenigen Azolylmethylcyclohexanole I, in denen der Rest D folgende Bedeutung hat:

$C_1$- bis $C_8$-Alkyl, vorzugsweise $C_1$- bis $C_4$-Alkyl, insbesondere Methyl, Ethyl, n-Propyl, Isopropyl, n-Butyl, sec.-Butyl und tert.-Butyl; von den $C_1$- bis $C_8$-Alkylresten, die mehr als 4 C-Atome aufweisen, sind als bevorzugt n-Pentyl und

Neopentyl zu nennen;

Phenyl und halogensubstituiertes Phenyl wie 2-Chlorphenyl, 3-Chlorphenyl, 4-Chlorphenyl, 2-Fluorphenyl, 3-Fluorphenyl, 4-Fluorphenyl sowie 2-Bromphenyl, 3-Bromphenyl und 4-Bromphenyl, 2,3-Dichlorphenyl, 2,4-Dichlorphenyl, 2,5-Dichlorphenyl und 2,6-Dichlorphenyl sowie 2-Chlor-4-fluorphenyl und 2-Chlor-6-fluorphenyl;

einfach durch Nitro-, Phenoxy-, Amino und $C_1$- bis $C_4$-Alkylgruppen substituiertes Phenyl, wie 3-Nitrophenyl, 4-Nitrophenyl, 3-Phenoxyphenyl, 4-Phenoxyphenyl, 3-Aminophenyl und 4-Aminophenyl sowie 4-Ethylphenyl, 4-Isopropylphenyl und 4-tert.-Butylphenyl;

durch zwei oder drei der bereits erwähnten, aber verschiedenartigen Reste substituiertes Phenyl, wie 2-Chlor-6-methylphenyl;

einfach oder zweifach durch $C_1$-$C_4$-Alkoxygruppen substituiertes Phenyl, wie 2-Methoxyphenyl, 3-Methoxyphenyl, 4-Methoxyphenyl, 4-tert.-Butyloxyphenyl und 2,4-Dimethoxyphenyl sowie 3,4-Dimethoxyphenyl;

dreifach halogensubstituiertes Methylphenyl, wie 2-Trifluormethyl-, 3-Trifluormethyl- und 4-Trifluormethylphenyl; p-Biphenylyl;

1-Naphthyl und 2-Naphthyl;
Heteroaryl mit 5 oder 6 Ringatomen, wobei 6-Ringe mit bis zu drei Stickstoffatomen, wie 2-Pyridyl, 3-Pyridyl und 4-Pyridyl insbesondere zu nennen sind, sowie 5-Ringe mit bevorzugt einem oder zwei der Heteroatome O, S und N, insbesondere 2-Furyl, 2-Thienyl, 3-Thienyl, 4-Oxazolyl, 4-Thiazolyl, 4-Isoxazolyl, 5-Isoxazolyl und 5-Imidazolyl;
$C_3$- bis $C_6$-Cycloalkyl, vorzugsweise Cyclopentyl und Cyclohexyl, $C_5$- bis $C_8$-Cycloalkenyl, vorzugsweise 3-Cyclohexenyl.

Die Reste A und B können unabhängig voneinander gleich oder verschieden sein, sie stehen vorzugsweise jeweils für ein Wasserstoffatom oder einen n-Alkylrest mit 1 bis 4 C-Atomen, insbesondere Methyl.

Als Säureadditionssalze eignen sich die pflanzenverträglichen Salze von solchen Säuren, welche die fungizide Wirkung von I nicht beeinträchtigen, also z.B. die Hydrochloride, Bromide, Sulfate, Nitrate, Phosphate, Oxalate und Dodecylbenzolsulfonate. Da die Wirksamkeit der Salze jedoch auf das Kation zurückgeht, kommt es auf das Anion i.a. nicht an. Die erfindungsgemäßen Wirkstoffsalze werden zweckmäßigerweise durch Umsetzung der Azolylmethylcyclohexanole (I) mit geeigneten Säuren hergestellt.

Metallkomplexe der erfindungsgemäßen Verbindungen I oder ihrer Salze werden bevorzugt mit Metallen der II. Hauptgruppe wie Magnesium oder Calcium, der III. und IV. Hauptgruppe wie Aluminium, Zinn oder Blei oder mit Metallen der I. bis VIII. Nebengruppe gebildet, wobei die Nebengruppenelemente der 4. Periode besonders bevorzugt sind, insbesondere Kupfer, Zink, Mangan, Eisen, Kobalt und Nickel. Hierzu werden die Azolylmethylcycloalkanole mit entsprechenden Metallsalzen umgesetzt.

Die Verbindungen der Formel I können hergestellt werden, indem man eine Verbindung der Formel II

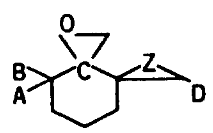

II

in welcher A, B, D und Z die oben angegebene Bedeutung haben, mit einer Verbindung der Formel III

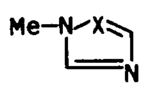

III

Me ist Wasserstoff oder ein Metallatom, X ist CH oder N
zur Umsetzung bringt. Von den Verbindungen III sind diejenigen bevorzugt, in denen Me für ein Wasserstoffatom oder ein Alkalimetallatom, insbesondere Natrium oder Kalium steht.

Falls Me ein Wasserstoffatom bedeutet, wird zweckmäßigerweise ein Gewichtsverhältnis III:II von 2:1 bis 6:1 eingehalten, insbesondere etwa 3:1.

Die Reaktion erfolgt gegebenenfalls in Gegenwart eines Lösungs- oder Verdünnungsmittels, zweckmäßig unter Zusatz einer anorganischen oder organischen Base und gegebenenfalls unter Zusatz eines Reaktionsbeschleunigers. Zu den bevorzugten Lösungs- und Verdünnungsmitteln gehörten Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, Sulfolan oder entsprechende Gemische.

Geeignete Basen, die gegebenenfalls auch als säurebindende Mittel bei der Reaktion verwendet werden können, sind beispielsweise Alkalihydroxide wie Lithium-, Natrium- oder Kaliumhydroxid, Alkalicarbonate wie Natrium- oder Kaliumcarbonat oder Natrium- oder Kaliumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumjodid, oder Kaliumjodid, quaternäre Ammoniumsalze wie Tetrabutylammoniumchlorid, -bromid oder -jodid oder Kronenether wie 12-Krone-4, 15-Krone-5, 18-Krone-6 oder Dicyclohexano-18-Krone-6 in Frage.

Die Umsetzung wird im allgemeinen bei Temperaturen zwischen 20 und 120 °C, drucklos oder unter Druck, kontinuierlich oder diskontinuierlich durchgeführt.

Steht Me für ein Metallatom, so wird ein Gewichtsverhältnis III:II von 1:1 bis 3:1, insbesondere 1:1 bevorzugt. Die Reaktion erfolgt gegebenenfalls in Gegenwart eines Lösungs- oder Verdünnungsmittels und gegebenenfalls unter Zusatz einer anorganischen oder organischen Base. Zu den bevorzugten Lösungs- und Verdünnungsmitteln gehören Amide wie Dimethylformamid, Diethylformamid, Dimethylacetamid, N-Methylpyrrolidon, Hexamethylphosphortriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butylat.

Man arbeitet im allgemeinen zwischen -10 und 120 °C, vorzugsweise bei 20 bis 80 °C. Bei Anwesenheit eines Lösungsmittels wird zweckmäßig beim Siedepunkt des jeweiligen Lösungsmittels gearbeitet.

Die Ausgangsverbindungen II lassen sich nach bekannten Methoden in einfacher Weise aus den Ketonen der Formel IV

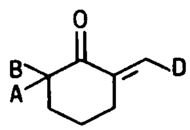

IV

herstellen, z.B. durch Umsetzung mit Trimethylsulfoxoniumjodid (vgl. Corey, Chaykovsky, J.Am.Chem.Soc.1962,64,3782).

Die Verbindungen IV lassen sich entsprechend allgemein bekannten Verfahren zur Olefinsynthese (Houben-Weyl-Müller, Methoden der organischen Chemie, Georg Thieme Verlag, Stuttgart, 1972, Bd. V, 1b) herstellen.

Beispiele für die Verbindungen II sind in Tabelle 1 aufgeführt.

Die Verbindungen der allgemeinen Formel I sowie ihre Salze und Metallkomplexe eignen sich als Fungizide bei guter Pflanzenverträglichkeit.

Herstellungsbeispiele

I. Herstellung der Ausgangsstoffe

Vorschrift 1

2-(4-Trifluormethyl-benzyliden)-cyclohexanon

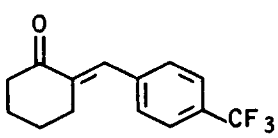

6 g Nickelacetylacetonat werden in 530 g (5,4 mol) Cyclohexanon gegeben und für 10 Minuten unter Rückfluß erhitzt. Anschließend werden 157 g (0,9 mol) 4-Trifluormethylbenzaldehyd langsam zugetropft und unter Auskreisung des Reaktionswassers am Rückfluß erhitzt. Danach wird das Reaktionsgemisch eingeengt und der verbleibende Rückstand im Vakuum destilliert, wobei bei 0,25 mbar und 114 °C Übergangstemperatur 75 g (33 %) Produkt erhalten werden.

Vorschrift 2

1-Oxa-2-(4-trifluormethylphenyl)-dispiro[2.0.2.4]decan

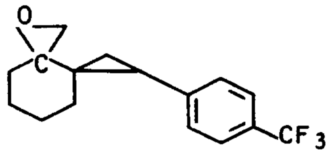

24 g 2-(4-Trifluormethylbenzyliden)-cyclohexanon werden in 100 ml N,N-Dimethylformamid gelöst und bei 0 °C unter Stickstoffatmosphäre mit 41,6 g (0,19 mol) Trimethylsulfoxoniumjodid versetzt.

Anschließend wird schnell 22 g Kalium-tert.-butylat zugegeben und auf 60 °C erhitzt. Nachdem das Reaktionsgemisch drei Tage bei 60 °C rührte, wird der Lösung 100 ml Wasser zugesetzt und mehrmals mit Methyl-tert.-butylether ausgeschüttelt. Die isolierte organische Phase wird zweimal mit Wasser gewaschen, daraufhin über Natriumsulfat getrocknet und eingeengt, wobei 21,2 g (80 %) Produkt erhalten werden.

II. Herstellung der Endprodukte

Beispiel 1

4-(1,2,4-Triazolyl-2-methyl)-4-hydroxy-1-(4-trifluormethylphenyl)spiro-[2.5]-octan.

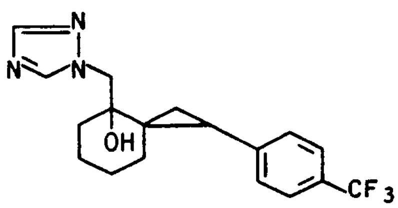

Eine Lösung von 18,2 g (0,26 mol) 1,2,4-Triazol in 100 ml N,N-Dimethylformamid wird mit 21 g Natronlauge (50 gew.-%ig) versetzt und für 30 Minuten auf 50 °C erwärmt. Anschließend werden bei Raumtemperatur 21 g (0,07 mol) 1-Oxa-2-(4-trifluormethylphenyl)-dispiro[2.0.2.4]octan, das in 50 ml N,N-Dimethylformamid gelöst ist, zugetropft. Nachdem das Reaktionsgemisch 15 Stunden bei Raumtemperatur (20 °C) gerührt worden war, wird der Lösung 100 ml Wasser zugesetzt und mehrmals mit Methyl-tert.-butylether ausgeschüttelt. Die isolierte organische Phase wird zweimal mit Wasser gewaschen, daraufhin über Natriumsulfat getrocknet und eingeengt. Durch Kristallisation des Rückstandes aus Methyl-tert.-butylether/n-Hexan erhält man das Produkt als Enantiomerengemisch. Ausbeute: 2,1 g (8 %)
Schmp.: 120 - 126 °C
Entsprechend Beispiel 1 können die in der Tabelle 2 aufgeführten Verbindungen hergestellt werden.

Tabelle 1

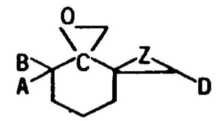

| Bsp | A | B | D | Z | $^1H$-NMR/Schmp |
|---|---|---|---|---|---|
| 1 | H | H | $4-CF_3-C_6H_4$ | $CH_2$ | 2,84 (dd) |
| 2 | H | H | $4-CF_3-C_6H_4$ | O | |
| 3 | H | $CH_3$ | $4-CF_3-C_6H_4$ | $CH_2$ | |
| 4 | $CH_3$ | $CH_3$ | $4-CF_3-C_6H_4$ | $CH_2$ | |
| 5 | $CH_3$ | $CH_3$ | $4-CF_3-C_6H_4$ | O | |
| 6 | H | H | $C_6H_5$ | $CH_2$ | |
| 7 | H | H | $C_6H_5$ | O | |
| 8 | $CH_3$ | $CH_3$ | $C_6H_5$ | $CH_2$ | |
| 9 | $CH_3$ | $CH_3$ | $C_6H_5$ | O | |
| 10 | H | H | $2-Cl-C_6H_4$ | $CH_2$ | |
| 11 | H | H | $2-Cl-C_6H_4$ | O | |
| 12 | $CH_3$ | $CH_3$ | $2-Cl-C_6H_4$ | $CH_2$ | |
| 13 | $CH_3$ | $CH_3$ | $2-Cl-C_6H_4$ | O | |
| 14 | H | H | $4-Cl-C_6H_4$ | $CH_2$ | |
| 15 | H | H | $4-Cl-C_6H_4$ | O | |
| 16 | $CH_3$ | $CH_3$ | $4-Cl-C_6H_4$ | $CH_2$ | |
| 17 | $CH_3$ | $CH_3$ | $4-Cl-C_6H_4$ | O | |
| 18 | H | H | $2,4-Cl_2-C_6H_3$ | $CH_2$ | |

Tabelle 1 Forts.

| Bsp | A | B | D | Z | $^1H$-NMR/Schmp |
|---|---|---|---|---|---|
| 43 | H | H | 2-Furyl | O | |
| 44 | $CH_3$ | $CH_3$ | 2-Furyl | $CH_2$ | |
| 45 | $CH_3$ | $CH_3$ | 2-Furyl | O | |
| 46 | H | H | 2-Thienyl | O | |
| 47 | H | H | 2-Thienyl | $CH_2$ | |
| 48 | $CH_3$ | $CH_3$ | 2-Thienyl | O | |
| 49 | H | H | 3-Pyridyl | $CH_2$ | |
| 50 | H | H | 3-Pyridyl | O | |
| 51 | $CH_3$ | $CH_3$ | 3-Pyridyl | $CH_2$ | |
| 52 | $CH_3$ | $CH_3$ | 3-Pyridyl | O | |
| 53 | H | H | tert.-$C_4H_9$ | $CH_2$ | |
| 54 | H | H | tert.-$C_4H_9$ | O | |
| 55 | $CH_3$ | $CH_3$ | tert.-$C_4H_9$ | $CH_2$ | |
| 56 | $CH_3$ | $CH_3$ | tert.-$C_4H_9$ | O | |
| 57 | H | H | Cyclohexyl | $CH_2$ | |
| 58 | H | H | Cyclohexyl | O | |
| 59 | $CH_3$ | $CH_3$ | Cyclohexyl | $CH_2$ | |
| 60 | $CH_3$ | $CH_3$ | Cyclohexyl | O | |

Tabelle 1 Forts.

| Bsp | A | B | D | Z | $^1H$-NMR/Schmp |
|---|---|---|---|---|---|
| 43 | H | H | 2-Furyl | O | |
| 44 | $CH_3$ | $CH_3$ | 2-Furyl | $CH_2$ | |
| 45 | $CH_3$ | $CH_3$ | 2-Furyl | O | |
| 46 | H | H | 2-Thienyl | O | |
| 47 | H | H | 2-Thienyl | $CH_2$ | |
| 48 | $CH_3$ | $CH_3$ | 2-Thienyl | O | |
| 49 | H | H | 3-Pyridyl | $CH_2$ | |
| 50 | H | H | 3-Pyridyl | O | |
| 51 | $CH_3$ | $CH_3$ | 3-Pyridyl | $CH_2$ | |
| 52 | $CH_3$ | $CH_3$ | 3-Pyridyl | O | |
| 53 | H | H | tert.-$C_4H_9$ | $CH_2$ | |
| 54 | H | H | tert.-$C_4H_9$ | O | |
| 55 | $CH_3$ | $CH_3$ | tert.-$C_4H_9$ | $CH_2$ | |
| 56 | $CH_3$ | $CH_3$ | tert.-$C_4H_9$ | O | |
| 57 | H | H | Cyclohexyl | $CH_2$ | |
| 58 | H | H | Cyclohexyl | O | |
| 59 | $CH_3$ | $CH_3$ | Cyclohexyl | $CH_2$ | |
| 60 | $CH_3$ | $CH_3$ | Cyclohexyl | O | |

Tabelle 2

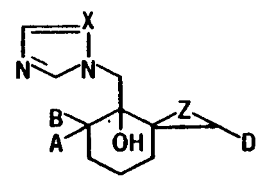

| Bsp | A | B | D | Z | X | Schmp/IR |
|---|---|---|---|---|---|---|
| 1.1 | H | H | $4-CF_3-C_6H_4$ | $CH_2$ | N | 120 - 126 °C |
| 1.2 | H | H | $4-CF_3-C_6H_4$ | O | N | |
| 1.3 | $CH_3$ | $CH_3$ | $4-CF_3-C_6H_4$ | $CH_2$ | N | |
| 1.4 | $CH_3$ | $CH_3$ | $4-CF_3-C_6H_4$ | O | N | |
| 1.5 | H | H | $C_6H_5$ | $CH_2$ | N | |
| 1.6 | H | H | $C_6H_5$ | O | N | |
| 1.7 | $CH_3$ | H | $C_6H_5$ | $CH_2$ | N | |
| 1.8 | $CH_3$ | H | $C_6H_5$ | O | N | |
| 1.9 | $CH_3$ | $CH_3$ | $C_6H_5$ | $CH_2$ | N | |
| 1.10 | $CH_3$ | $CH_3$ | $C_6H_5$ | O | N | |
| 1.11 | H | tert.-$C_4H_9$ | $C_6H_5$ | O | N | |
| 1.12 | H | H | $2-Cl-C_6H_4$ | $CH_2$ | N | |
| 1.13 | H | H | $2-Cl-C_6H_4$ | O | N | |
| 1.14 | $CH_3$ | $CH_3$ | $2-Cl-C_6H_4$ | $CH_2$ | N | |
| 1.15 | $CH_3$ | $CH_3$ | $2-Cl-C_6H_4$ | O | N | |

Tabelle 2 Forts.

| Bsp | A | B | D | Z | X | Schmp/IR |
|---|---|---|---|---|---|---|
| 1.16 | $CH_3$ | $CH_3$ | $2\text{-}Cl\text{-}C_6H_4$ | $CH_2$ | CH | |
| 1.17 | $CH_3$ | $CH_3$ | $2\text{-}Cl\text{-}C_6H_4$ | O | CH | |
| 1.18 | H | H | $4\text{-}Cl\text{-}C_6H_4$ | $CH_2$ | N | |
| 1.19 | H | H | $4\text{-}Cl\text{-}C_6H_4$ | O | N | |
| 1.20 | H | H | $4\text{-}Cl\text{-}C_6H_4$ | $CH_2$ | CH | |
| 1.21 | H | H | $4\text{-}Cl\text{-}C_6H_4$ | O | CH | |
| 1.22 | $CH_3$ | H | $4\text{-}Cl\text{-}C_6H_4$ | $CH_2$ | N | |
| 1.23 | $CH_3$ | H | $4\text{-}Cl\text{-}C_6H_4$ | O | N | |
| 1.24 | $CH_3$ | $CH_3$ | $4\text{-}Cl\text{-}C_6H_4$ | O | N | |
| 1.25 | $CH_3$ | $CH_3$ | $4\text{-}Cl\text{-}C_6H_4$ | $CH_2$ | N | |
| 1.26 | $CH_3$ | $CH_3$ | $4\text{-}Cl\text{-}C_6H_4$ | O | CH | |
| 1.27 | $CH_3$ | $CH_3$ | $4\text{-}Cl\text{-}C_6H_4$ | $CH_2$ | CH | |
| 1.28 | H | $C_2H_5$ | $4\text{-}Cl\text{-}C_6H_4$ | O | N | |
| 1.29 | H | $C_3H_7$ | $4\text{-}Cl\text{-}C_6H_4$ | O | N | |
| 1.30 | H | iso-$C_3H_7$ | $4\text{-}Cl\text{-}C_6H_4$ | O | N | |
| 1.31 | H | H | $2,4\text{-}Cl_2\text{-}C_6H_3$ | $CH_2$ | N | |
| 1.32 | H | H | $2,4\text{-}Cl_2\text{-}C_6H_3$ | O | N | 206 - 212 °C |
| 1.33 | H | H | $2,4\text{-}Cl_2\text{-}C_6H_3$ | $CH_2$ | CH | |
| 1.34 | H | H | $2,4\text{-}Cl_2\text{-}C_6H_3$ | O | CH | > 250 °C |
| 1.35 | $CH_3$ | H | $2,4\text{-}Cl_2\text{-}C_6H_3$ | $CH_2$ | N | |
| 1.36 | $CH_3$ | H | $2,4\text{-}Cl_2\text{-}C_6H_3$ | O | N | |
| 1.37 | $CH_3$ | $CH_3$ | $2,4\text{-}Cl_2\text{-}C_6H_3$ | $CH_2$ | N | |
| 1.38 | $CH_3$ | $CH_3$ | $2,4\text{-}Cl_2\text{-}C_6H_3$ | O | N | |
| 1.39 | H | H | $2\text{-}F\text{-}C_6H_4$ | $CH_2$ | N | |
| 1.40 | H | H | $2\text{-}F\text{-}C_6H_4$ | O | N | |

Tabelle 2 Forts.

| Bsp | A | B | D | Z | X | Schmp/IR |
|---|---|---|---|---|---|---|
| 1.41 | $CH_3$ | H | 2-F-$C_6H_4$ | $CH_2$ | N | |
| 1.42 | $CH_3$ | H | 2-F-$C_6H_4$ | O | N | |
| 1.43 | $CH_3$ | $CH_3$ | 2-F-$C_6H_4$ | $CH_2$ | N | |
| 1.44 | $CH_3$ | $CH_3$ | 2-F-$C_6H_4$ | O | N | |
| 1.45 | H | H | 4-F-$C_6H_4$ | $CH_2$ | N | |
| 1.46 | H | H | 4-F-$C_6H_4$ | O | N | |
| 1.47 | H | H | 4-F-$C_6H_4$ | $CH_2$ | CH | |
| 1.48 | H | H | 4-F-$C_6H_4$ | O | CH | |
| 1.49 | $CH_3$ | H | 4-F-$C_6H_4$ | $CH_2$ | N | |
| 1.50 | $CH_3$ | H | 4-F-$C_6H_4$ | O | N | |
| 1.51 | $CH_3$ | $CH_3$ | 4-F-$C_6H_4$ | $CH_2$ | N | |
| 1.52 | $CH_3$ | $CH_3$ | 4-F-$C_6H_4$ | O | N | 2941,1512,1276,1224 $cm^{-1}$ |
| 1.53 | $CH_3$ | $CH_3$ | 4-F-$C_6H_4$ | $CH_2$ | CH | |
| 1.54 | $CH_3$ | $CH_3$ | 4-F-$C_6H_4$ | O | CH | |
| 1.55 | H | $C_2H_5$ | 4-F-$C_6H_4$ | O | N | |
| 1.56 | H | $C_3H_7$ | 4-F-$C_6H_4$ | O | N | |
| 1.57 | H | $C_4H_9$ | 4-F-$C_6H_4$ | O | N | |
| 1.58 | H | $C_5H_{11}$ | 4-F-$C_6H_4$ | O | N | |
| 1.59 | H | $C_6H_{13}$ | 4-F-$C_6H_4$ | O | N | |
| 1.60 | H | H | 2-Br-$C_6H_4$ | $CH_2$ | N | |
| 1.61 | H | H | 2-Br-$C_6H_4$ | O | N | |
| 1.62 | $CH_3$ | $CH_3$ | 2-Br-$C_6H_4$ | $CH_2$ | N | |
| 1.63 | $CH_3$ | $CH_3$ | 2-Br-$C_6H_4$ | O | N | |
| 1.64 | H | H | 4-Br-$C_6H_4$ | $CH_2$ | N | |
| 1.65 | H | H | 4-Br-$C_6H_4$ | O | N | |
| 1.66 | $CH_3$ | H | 4-Br-$C_6H_4$ | $CH_2$ | N | |

Tabelle 2 Forts.

| Bsp | A | B | D | Z | X | Schmp/IR |
|---|---|---|---|---|---|---|
| 1.67 | $CH_3$ | $CH_3$ | $4-Br-C_6H_4$ | O | N | |
| 1.68 | H | H | $2-CH_3-C_6H_4$ | $CH_2$ | N | |
| 1.69 | H | H | $2-CH_3-C_6H_4$ | O | N | |
| 1.70 | $CH_3$ | $CH_3$ | $2-CH_3-C_6H_4$ | $CH_2$ | N | |
| 1.71 | $CH_3$ | $CH_3$ | $2-CH_3-C_6H_4$ | O | N | |
| 1.72 | H | H | $4-CH_3-C_6H_4$ | $CH_2$ | N | |
| 1.73 | H | H | $4-CH_3-C_6H_4$ | O | N | |
| 1.74 | $CH_3$ | $CH_3$ | $4-CH_3-C_6H_4$ | $CH_2$ | N | |
| 1.75 | $CH_3$ | $CH_3$ | $4-CH_3-C_6H_4$ | O | N | |
| 1.76 | H | H | $2-CF_3-C_6H_4$ | $CH_2$ | N | |
| 1.77 | H | H | $2-CF_3-C_6H_4$ | O | N | |
| 1.78 | $CH_3$ | $CH_3$ | $2-CF_3-C_6H_4$ | $CH_2$ | N | |
| 1.79 | $CH_3$ | $CH_3$ | $2-CF_3-C_6H_4$ | O | N | |
| 1.80 | H | H | $2-OCH_3-C_6H_4$ | $CH_2$ | N | 169 - 173 °C |
| 1.81 | H | H | $2-OCH_3-C_6H_4$ | O | N | |
| 1.82 | H | H | $2-OCH_3-C_6H_4$ | $CH_2$ | CH | 142 - 145 °C |
| 1.83 | H | H | $2-OCH_3-C_6H_4$ | O | CH | |
| 1.84 | $CH_3$ | $CH_3$ | $2-OCH_3-C_6H_4$ | $CH_2$ | N | |
| 1.85 | $CH_3$ | $CH_3$ | $2-OCH_3-C_6H_4$ | O | N | |
| 1.86 | H | H | $4-OCH_3-C_6H_4$ | $CH_2$ | N | |
| 1.87 | H | H | $4-OCH_3-C_6H_4$ | O | N | |
| 1.88 | $CH_3$ | $CH_3$ | $4-OCH_3-C_6H_4$ | $CH_2$ | N | |
| 1.89 | $CH_3$ | $CH_3$ | $4-OCH_3-C_6H_4$ | O | N | |
| 1.90 | H | H | $3-NO_2-C_6H_4$ | $CH_2$ | N | |
| 1.91 | H | H | $3-NO_2-C_6H_4$ | O | N | |

Tabelle 2 Forts.

| Bsp | A | B | D | Z | X | Schmp/IR |
|---|---|---|---|---|---|---|
| 1.92 | H | H | 3-$NH_2$-$C_6H_4$ | $CH_2$ | N | |
| 1.93 | H | H | 3-$NH_2$-$C_6H_4$ | O | N | |
| 1.94 | H | H | $C_6H_5$-O-$C_6H_4$ | $CH_2$ | N | |
| 1.95 | H | H | $C_6H_5$-O-$C_6H_4$ | O | N | |
| 1.96 | H | H | 1-Naphthyl | $CH_2$ | N | |
| 1.97 | H | H | 1-Naphthyl | O | N | |
| 1.98 | H | H | 2-Naphthyl | $CH_2$ | N | |
| 1.99 | H | H | 2-Naphthyl | O | N | |
| 1.100 | $CH_3$ | $CH_3$ | 2-Naphthyl | O | N | |
| 1.101 | $CH_3$ | $CH_3$ | 2-Naphthyl | $CH_2$ | N | |
| 1.102 | H | H | 4-Biphenylyl | $CH_2$ | N | |
| 1.103 | H | H | 4-Biphenylyl | O | N | |
| 1.104 | $CH_3$ | $CH_3$ | 4-Biphenylyl | $CH_2$ | N | |
| 1.105 | $CH_3$ | $CH_3$ | 4-Biphenylyl | O | N | |
| 1.106 | H | H | tert.-$C_4H_9$ | $CH_2$ | N | |
| 1.107 | H | H | tert.-$C_4H_9$ | O | N | |
| 1.108 | $CH_3$ | $CH_3$ | tert.-$C_4H_9$ | $CH_2$ | N | |
| 1.109 | $CH_3$ | $CH_3$ | tert.-$C_4H_9$ | O | N | |
| 1.110 | H | H | 3-Cyclohexenyl | $CH_2$ | N | |
| 1.111 | H | H | 3-Cyclohexenyl | O | N | |
| 1.112 | $CH_3$ | $CH_3$ | 3-Cyclohexenyl | $CH_2$ | N | |
| 1.113 | $CH_3$ | $CH_3$ | 3-Cyclohexenyl | O | N | |
| 1.114 | H | H | Cyclopentyl | O | N | |
| 1.115 | H | H | Cyclopentyl | $CH_2$ | N | |

Tabelle 2 Forts.

| Bsp | A | B | D | Z | X | Schmp/IR |
|---|---|---|---|---|---|---|
| 1.116 | $CH_3$ | $CH_3$ | Cyclopentyl | O | N | |
| 1.117 | $CH_3$ | $CH_3$ | Cyclopentyl | $CH_2$ | N | |
| 1.118 | $CH_3$ | $CH_3$ | Cyclopentyl | O | CH | |
| 1.119 | $CH_3$ | $CH_3$ | Cyclopentyl | $CH_2$ | CH | |
| 1.120 | H | H | Cyclohexyl | O | N | |
| 1.121 | H | H | Cyclohexyl | $CH_2$ | N | |
| 1.122 | H | H | Cyclohexyl | O | CH | |
| 1.123 | H | H | Cyclohexyl | $CH_2$ | CH | |
| 1.124 | $CH_3$ | $CH_3$ | Cyclohexyl | O | N | |
| 1.125 | $CH_3$ | $CH_3$ | Cyclohexyl | $CH_2$ | N | |

Die neuen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin), Dimethylformamid und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen der Verbindung Nr. 1.1 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 1.32, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 1.34, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 1.52, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 1.80, 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 1.82 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 1.1, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 1.32, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 1.34, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydkondensates und 68 Gew.-Teilen eines paraffinischen Mineralöls.

Die Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Anwendungsbeispiele

Als Vergleichssubstanz wurde E-1-[1-(4-Chlorphenyl)-1-hydroxy-2-(1,2,4-triazol-1-yl)-1-ethyl]-2-phenylcyclopropan (A) - bekannt aus EP 212 605 - verwendet.

Anwendungsbeispiel 1

Wirksamkeit gegen Pyricularie oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24°C und 95 bis 99 % relativer Luftfeuchtigkeit aufgestellt. Nach 6 Tagen wurde das Ausmaß des Krankheitsbefalls ermittelt.

Das Ergebnis des Versuches zeigt, daß die Wirkstoffe Nr. 1.52 und 1.81 bei der Anwendung als 500 ppm Wirkstoff enthaltende wäßrige Dispersion eine bessere fungizide Wirkung zeigen (90 %) als der bekannte Vergleichswirkstoff A (50 %).

Anwendungsbeispiel 2

Wirksamkeit gegen Botrytis cinerea

Paprikasämlinge der Sorte "Neusiedler Ideal Elite" wurden, nachdem sich 4 bis 5 Blätter, gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedecken.

Das Ergebnis des Versuchs zeigt, daß der Wirkstoff 1.52 bei der Anwendung als 500 ppm Wirkstoff enthaltende wäßrige Dispersion eine bessere fungizide Wirkung hat (95 %) als der bekannte Vergleichswirkstoff A (40 %)

## Patentansprüche

**1.** Azolylmethylspiro-[2.5]-octanole der allgemeinen Formel I

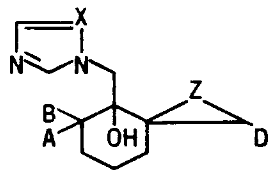

in welcher
A und B
jeweils für ein Wasserstoffatom oder eine $C_1$- bis $C_4$-Alkylgruppe stehen;
D
$C_1$- bis $C_8$-Alkyl, $C_3$- bis $C_6$-Cycloalkyl, $C_5$- bis $C_8$-Cycloalkenyl, Biphenylyl, Naphthyl, Heteroaryl oder Phenyl bedeutet, wobei jeder dieser Reste einfach bis dreifach durch Halogen, Nitro, Phenoxy, Amino, $C_1$- bis $C_4$-Alkyl, $C_1$- bis $C_4$-Alkoxy oder $C_1$- bis $C_4$-Halogenalkyl substituiert sein kann;
Z
den Rest $CH_2$ oder O bedeutet,
X
den Rest CH oder N bedeutet, sowie deren pflanzenverträgliche Säureadditionssalze und Metallkomplexe.

**2.** Azolylmethylspiro-[2.5]-octanole der allgemeinen Formel I gemäß Anspruch 1, in der D einen gegebenenfalls einfach bis dreifach durch Fluor oder Chlor substituierten Phenylrest bedeutet.

**3.** Verfahren zur Herstellung von Azolylmethylspiro-[2.5]-octanolen der Formel I

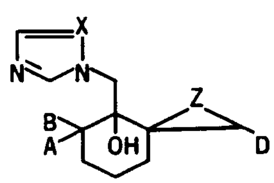

in welcher
A und B
jeweils für ein Wasserstoffatom oder eine $C_1$- bis $C_4$-Alkylgruppe stehen;
D
$C_1$- bis $C_8$-Alkyl, $C_3$- bis $C_6$-Cycloalkyl, $C_5$- bis $C_8$-Cycloalkenyl, Biphenylyl, Naphthyl, Heteroaryl oder Phenyl bedeutet, wobei jeder dieser Reste einfach bis dreifach durch Halogen, Nitro, Phenoxy, Amino,

$C_1$- bis $C_4$-Alkyl, $C_1$- bis $C_4$-Alkoxy oder $C_1$- bis $C_4$-Halogenalkyl substituiert sein kann;
Z
den Rest $CH_2$ oder O bedeutet,
X
den Rest CH oder N bedeutet, sowie deren pflanzenverträgliche Säureadditionssalze und Metallkomplexe, dadurch gekennzeichnet, daß man eine Verbindung der Formel II

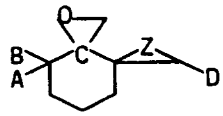

II

in welcher A, B, D und Z die oben angegebene Bedeutung haben, mit einer Verbindung der allgemeinen Formel III

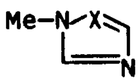

III

in welcher Me für ein Wasserstoff- oder ein Metallatom steht und X, CH oder N bedeutet, umsetzt und die so erhaltenen Verbindungen gegebenenfalls mit pflanzenverträglichen Säuren in ihre Salze oder in ihre Metallkomplexe überführt.

**4.** Verbindungen der allgemeinen Formel II

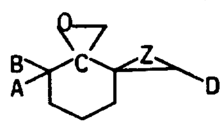

in der A, B, D und Z die in Anspruch 1 genannten Bedeutungen haben.

**5.** Fungizides Mittel, enthaltend einen Trägerstoff und eine fungizid wirksame Menge eines Azolylmethylspiro-[2.5]-octanols der allgemeinen Formel I,

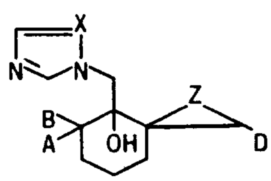

in welcher
A und B
jeweils für ein Wasserstoffatom oder eine $C_1$- bis $C_4$-Alkylgruppe stehen,
D
$C_1$- bis $C_8$-Alkyl, $C_3$- bis $C_6$-Cycloalkyl, $C_5$- bis $C_8$-Cycloalkenyl, Biphenylyl, Naphthyl, Heteroaryl oder Phenyl bedeutet, wobei jeder dieser Reste einfach bis dreifach durch Halogen, Nitro, Phenoxy, Amino, $C_1$- bis $C_4$-Alkyl, $C_1$- bis $C_4$-Alkoxy oder $C_1$- bis $C_4$-Halogenalkyl substituiert sein kann,
Z
den Rest $CH_2$ oder O bedeutet,
X
den Rest CH oder N bedeutet,
oder dessen pflanzenverträglichen Säureadditionssalzes oder dessen Metallkomplexes.

6. Verfahren zur Bekämpfung von Pilzen, dadurch gekennzeichnet, daß man eine fungizid wirksame Menge eines Azolylmethylspiro-[2.5]-octanols der allgemeinen Formel I

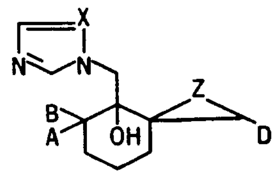

in welcher
A und B
jeweils für ein Wasserstoffatom oder eine $C_1$- bis $C_4$-Alkylgruppe stehen,
D
$C_1$- bis $C_8$-Alkyl, $C_3$- bis $C_6$-Cycloalkyl, $C_5$- bis $C_8$-Cycloalkenyl, Biphenylyl, Naphthyl, Heteroaryl oder Phenyl bedeutet, wobei jeder dieser Reste einfach bis dreifach durch Halogen, Nitro, Phenoxy, Amino, $C_1$- bis $C_4$-Alkyl, $C_1$- bis $C_4$-Alkoxy oder $C_1$- bis $C_4$-Halogenalkyl substituiert sein kann,
Z
den Rest $CH_2$ oder O bedeutet,
X
den Rest CH oder N bedeutet,
oder dessen pflanzenverträglichen Säureadditionssalzes oder Metallkomplexes auf Pilze oder durch Pilzbefall bedrohte Materialien, Flächen, Pflanzen oder Saatgüter einwirken läßt.

7. 4-(1,2,4-Triazolyl-1-ylmethyl)-4-hydroxy-1-(4-trifluormethylphenyl)spiro-[2.5]-octan.

## Claims

1. An azolylmethylspiro[2.5]octanol of the formula I

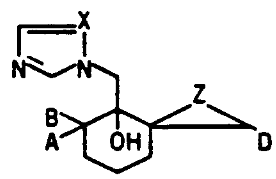

where
A and B are each hydrogen or $C_1$-$C_4$-alkyl;
D is $C_1$-$C_8$-alkyl, $C_3$-$C_6$-cycloalkyl, $C_5$-$C_8$-cycloalkenyl, biphenylyl, naphthyl, hetaryl or phenyl, it being possible for each of these radicals to be substituted once to three times by halogen, nitro, phenoxy, amino, $C_1$-$C_4$-alkyl, $C_1$-$C_4$-alkoxy or $C_1$-$C_4$-haloalkyl;
Z is $CH_2$ or O, and
X is CH or N, and the plant-compatible acid addition salts and metal complexes thereof.

2. An azolylmethylspiro[2.5]octanol of the formula I as claimed in claim 1, where D is phenyl which is unsubstituted or substituted once to three times by fluorine or chlorine.

**3.** A process for preparing an azolylmethylspiro[2.5]octanol of the formula I

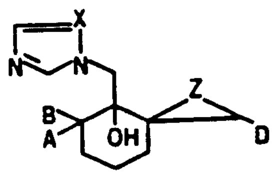

where
A and B are each hydrogen or $C_1$-$C_4$-alkyl;
D is $C_1$-$C_8$-alkyl, $C_3$-$C_6$-cycloalkyl, $C_5$-$C_8$-cycloalkenyl, biphenylyl, naphthyl, hetaryl or phenyl, it being possible for each of these radicals to be substituted once to three times by halogen, nitro, phenoxy, amino, $C_1$-$C_4$-alkyl, $C_1$-$C_4$-alkoxy or $C_1$-$C_4$-haloalkyl;
Z is $CH_2$ or O, and
X is CH or N, and the plant-compatible acid addition salts and metal complexes thereof, which comprises reacting a compound of the formula II

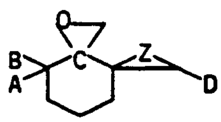

II

where A, B, D and Z have the abovementioned meanings, with a compound of the formula III

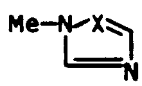

III

where Me is hydrogen or a metal atom, and X is CH or N, and converting the resulting compounds where appropriate with plant-compatible acids into the salts thereof or into the metal complexes thereof.

**4.** A compound of the formula II

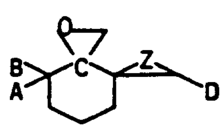

where A, B, D and Z have the meanings specified in claim 1.

**5.** A fungicidal agent containing a carrier and a fungicidal amount of an azolylmethylspiro[2.5]octanol of the formula I

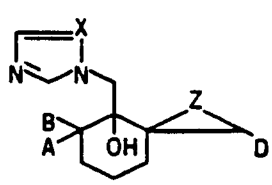

where

A and B are each hydrogen or $C_1$-$C_4$-alkyl;
D is $C_1$-$C_8$-alkyl, $C_3$-$C_6$-cycloalkyl, $C_5$-$C_8$-cycloalkenyl, biphenylyl, naphthyl, hetaryl or phenyl, it being possible for each of these radicals to be substituted once to three times by halogen, nitro, phenoxy, amino, $C_1$-$C_4$-alkyl, $C_1$-$C_4$-alkoxy or $C_1$-$C_4$-haloalkyl;
Z is $CH_2$ or O, and
X is CH or N, or the plant-compatible acid addition salt or metal complex thereof.

**6.** A method of controlling fungi, which comprises exposing fungi or materials, areas, plants or seeds threatened by fungal attack to a fungicidal amount of an azolylmethylspiro[2.5]octanol of the formula I

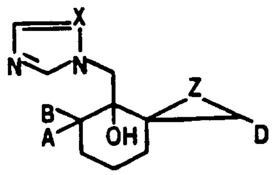

where
A and B are each hydrogen or $C_1$-$C_4$-alkyl;
D is $C_1$-$C_8$-alkyl, $C_3$-$C_6$-cycloalkyl, $C_5$-$C_8$-cycloalkenyl, biphenylyl, naphthyl, hetaryl or phenyl, it being possible for each of these radicals to be substituted once to three times by halogen, nitro, phenoxy, amino, $C_1$-$C_4$-alkyl, $C_1$-$C_4$-alkoxy or $C_1$-$C_4$-haloalkyl;
Z is $CH_2$ or O, and
X is CH or N, or the plant-compatible acid addition salt or metal complex thereof.

**7.** 4-(1,2,4-Triazol-1-ylmethyl)-4-hydroxy-1-(4-trifluoromethylphenyl)spiro[2.5]octane.

## Revendications

**1.** [Azolylméthylspiro-[2.5]-octanoles] de formule générale I

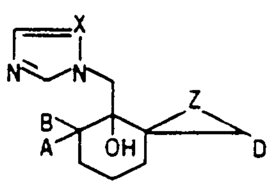

dans laquelle
A et B sont mis chacun par un atome d'hydrogène ou un groupe alkyle en C1 à C4;
D représente alkyle en C1 à C8, cycloalkyle C3 à C6, cycloalcényle C5 à C8, biphényle, naphtyle, hétéroaryle ou phényle, chacun des restes pouvant être substitué une à trois fois par halogène, nitro, phénoxy, amino, alkyle C1 à C4, alcoxy C1 à C4 ou halogénoalkyle C1 à C4;
Z représente le reste $CH_2$ ou O,
X représente le reste CH ou N,
ainsi que les sels d'addition d'acide et complexes métalliques acceptables pour les plantes.

**2.** [Azolylméthylspiro-[2.5]-octanoles] de formule générale I selon la revendication 1, dans laquelle D représente un reste phényle éventuellement substitué une à trois fois par fluor ou chlore.

**3.** Procédé de préparation de [azolylméthylspiro-[2.5]-octanoles] de formule générale I

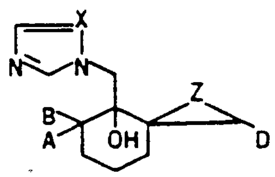

dans laquelle
A et B sont mis chacun par un atome d'hydrogène ou un groupe alkyle en C1 à C4;
D représente alkyle en C1 à C8, cycloalkyle C3 à C6, cycloalcényle C5 à C8, biphényle, naphtyle, hétéroaryle ou phényle, chacun des restes pouvant être substitué une à trois fois par halogène, nitro, phénoxy, amino, alkyle C1 à C4, alcoxy C1 à C4 ou halogénoalkyle C1 à C4;
Z représente le reste $CH_2$ ou O,
X représente le reste CH ou N,
ainsi que les sels d'addition d'acide et complexes métalliques acceptables pour les plantes, caractérisé par le fait que l'on fait réagir un composé de la formule II

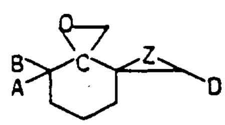

II

dans laquelle A, B, D et Z ont la signification sus-indiquée avec un composé de formule générale III

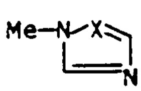

III

dans laquelle Me est mis par un atome d'hydrogène ou un atome de métal et X représente CH ou N et l'on transforme les composés ainsi obtenus, éventuellement avec des acides acceptables pour les plantes, en leurs sels ou en leurs complexes métalliques.

**4.** Composés de formule générale II

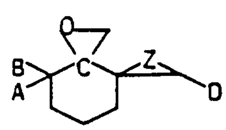

dans laquelle A, B, D et Z ont la signification indiquée dans la revendication 1.

**5.** Agent fongicide contenant un support et une quantité efficace du point de vue fongicide d'une [azolylméthylspiro-[2.5]-octanoles] de formule générale I

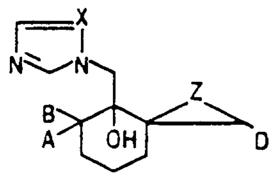

dans laquelle
A et B sont mis chacun par un atome d'hydrogène ou un groupe alkyle en C1 à C4;
D représente alkyle en C1 à C8, cycloalkyle C3 à C6, cycloalcényle C5 à C8, biphényle, naphtyle, hétéroaryle ou phényle, chacun des restes pouvant être substitué une à trois fois par halogène, nitro, phénoxy, amino, alkyle C1 à C4, alcoxy C1 à C4 ou halogénoalkyle C1 à C4;
Z représente le reste $CH_2$ ou O,
X représente le reste CH ou N,
ainsi que les sels d'addition d'acide et complexes métalliques acceptables pour les plantes.

6. Procédé de lutte contre les champignons, caractérisé par le fait que l'on fait agir sur les champignons ou sur les matériaux, surfaces, plantes ou semences menacés d'une attaque par champignons [azolylméthylspiro-[2.5]-octanoles] de formule générale I

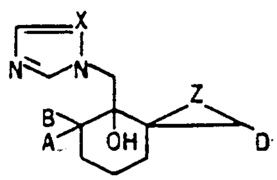

dans laquelle
A et B sont mis chacun par un atome d'hydrogène ou un groupe alkyle en C1 à C4;
D représente alkyle en C1 à C8, cycloalkyle C3 à C6, cycloalcényle C5 à C8, biphényle, naphtyle, hétéroaryle ou phényle, chacun des restes pouvant être substitué une à trois fois par halogène, nitro, phénoxy, amino, alkyle C1 à C4, alcoxy C1 à C4 ou halogénoalkyle C1 à C4;
Z représente le reste $CH_2$ ou O,
X représente le reste CH ou N, ainsi que les sels d'addition d'acide et complexes métalliques acceptables pour les plantes.

7. 4-(1,2,4-triazolyl-1-ylméthyle)-4-hydroxy-1-(4-trifluorométhylphényle)-spiro-[2.5]-octane.